# EUROPEAN PATENT SPECIFICATION

(11) **EP 3 856 249 B1**
(45) Date of publication and mention of the grant of the patent: **09.11.2022**
(21) Application number: 19787160.1
(22) Date of filing: 24.09.2019
(51) Int. Cl.: A61K 38/39, A61P 9/00

(54) **TREATMENT OF MYOCARDIAL INFARCTION**
MYOKARDINFARKTBEHANDLUNG
TRAITEMENT DE L'INFARCTUS DU MYOCARDE

(30) Priority: 26.09.2018 EP 18196891
(43) Date of publication of application: 04.08.2021
(73) Proprietor: National University of Ireland Galway, Galway (IE); Universidad de Valladolid, 47005 Valladolid (ES)
(72) Inventor: PANDIT, Abhay, Galway (IE); CONTESSOTTO, Paolo, Galway (IE); DA COSTA, Mark, Galway (IE); RODRIGUEZ-CABELLO, Jose Carlos, Valladolid (ES)
(74) Representative: Purdylucey Intellectual Property
(86) International application number: PCT/EP2019/075758
(87) International publication number: WO 2020/064767

(56) References cited:
- US-A1- 2018 078 577
- MAHARA ATSUSHI ET AL: "In vivo guided vascular regeneration with a non-porous elastin-like polypeptide hydrogel tubular scaffold", JOURNAL OF BIOMEDICAL MATERIALS RESEARCH. PART A,, vol. 105, 3 April 2017 (2017-04-03), pages 1746-1755, XP002789565, DOI: 10.1002/JBM.A.36018
- CASTELLANOS MARIA ISABEL ET AL: "Biofunctionalization of REDV elastin-like recombinamers improves endothelialization on CoCr alloy surfaces for cardiovascular applications", COLLOIDS AND SURFACES. B, BIOINTERFACES, ELSEVIER, AMSTERDAM, NL, vol. 127, 8 January 2015 (2015-01-08), pages 22-32, XP029211291, ISSN: 0927-7765, DOI: 10.1016/J.COLSURFB.2014.12.056
- KAMBE YUSUKE ET AL: "Cardiac differentiation of induced pluripotent stem cells on elastin-like protein-based hydrogels presenting a single-cell adhesion sequence", POLYMER JOURNAL, SOCIETY OF POLYMER SCIENCE, TOKYO, JP, vol. 51, no. 1, 3 August 2018 (2018-08-03) , pages 97-105, XP036657253, ISSN: 0032-3896, DOI: 10.1038/S41428-018-0110-2 [retrieved on 2018-08-03]

## Description

### Field of the Invention

The present invention relates to a hydrogel for use in treatment of myocardial infarction in a mammal.

### Background to the Invention

Myocardial infarction (MI) is commonly referred to as a heart attack. This describes the death (infarction) of a part of the heart muscle due to a sudden inadequacy of blood supply. The most frequent cause of MI by far is coronary artery disease (atherosclerotic heart disease). Coronary artery disease is the most common disease in the world, more common than cancer. Less often, MI may result from other causes of reduced myocardial blood flow, such as severe low blood pressure (hypotension) or prolonged spasm of a coronary artery (vasospasm).

Myocardial infarction may also result from conditions that drastically increase the heart's need for oxygen, such as severe hyperthyroidism. This section pertains primarily to MI caused by atherosclerotic coronary artery disease, a process in which fatty material (atherosclerotic plaques) is deposited in the walls of the coronary arteries supplying the heart muscle; this same process may also be occurring in arteries throughout the body. As the fatty deposits slowly increase in size over the years, this results in areas of narrowing (stenosis) inside the artery and reduced blood flow to the heart. The disease process produces no symptoms until stenosis becomes severe enough to result in an inadequate blood supply (ischemia) to meet the needs of the heart.

Myocardial ischemia that is mild to moderate in degree, and/or of brief duration, may cause chest pain (angina pectoris) but does not result in permanent tissue damage. Prolonged, severe myocardial ischemia results in tissue death which is a myocardial infarction.

The usual precipitating event for a Myocardial infarction is a rapid formation of a blood clot in an already narrowed coronary artery where plaque has ruptured. A clot may obstruct the artery where it forms, or it may be carried downstream until it obstructs a smaller artery. In either case, the portion of heart muscle supplied by the blocked artery is deprived of blood flow and undergoes infarction.

The location and size of a MI depend on the site(s) of arterial obstruction, and on whether there is overlapping blood supply from other coronary vessels (collateral circulation). The infarction may either involve the full thickness of the heart muscle (transmural) or a partial thickness (subendocardial).

Mahara et al (Journal of Biomedical Materials Research. Part A - Vol. 105 1746-1755 (2017)) discloses in-vivo guided vascular regeneration with a non-porous elastin-like polypeptide hydrogel tubular scaffold. Castellanos et al (Colloids and Surfaces. B, Biointerfaces - ISSN:0927-7765 - 2015) describes biofunctionalization of REDV elastin-like recombinamers and their use to improve endotheliasation on CoCr alloy surfaces for cardiovascular applications. Kambe et al (Polymer Journal, Society of Polymer Science, Vol. 51, No: 1 (2018)) describes cardiac differentiation of induced pluripotent stem cells on elastin-like protein-based hydrogels presenting a single-cell adhesion sequence.

### Summary of the Invention

The present invention addresses the need for a treatment of myocardial infarction that induces repair of cardiac muscle damaged by the infarct. The Applicant has discovered that delivery of an elastin hydrogel, and preferably a crosslinked elastin-like recombinamer (ELR) hydrogel, into the coronary muscle damaged by an acute myocardial infarction can modulate the response of the damaged coronary muscle, for example by limiting scarring, inducing positive remodelling of the damaged muscle, and/or restores cardiac muscle function to a clinically significant extent after infarction. The treatment generally involves administration of the hydrogel at two days, and preferably at least three, four, five, six, or seven days after the myocardial infarction, for example at least two to four days, and preferably at last five to seven days, after the myocardial infarction, as the delayed administration has been found to lead to better functional recovery and remodelling of the affected cardiac muscle. An injectable ELR is described herein, which is suitable for minimally invasive delivery by epicardial injection, and forms part of the invention. According to a first aspect of the present invention, there is provided an elastin hydrogel (i.e. a therapeutically effective amount of an elastin hydrogel), for use in a method of treating a mammal that has suffered a myocardial infarction to modulate the response of the cardiac muscle damaged by the infarct, in which the hydrogel is administered into the cardiac muscle damaged by the infarct preferably at least 48 hours after the myocardial infarction. This in effect limits scarring, induces positive remodelling of the damaged muscle and/or restores cardiac muscle function to a significant extent after infarction.

In one embodiment, the elastin hydrogel is an elastin-like recombinamer (ELR) hydrogel.

In one embodiment, the hydrogel is administered into the cardiac muscle damaged by the infarct preferably at least 72 or 96 hours after the myocardial infarction. In one embodiment, the hydrogel is administered into the cardiac muscle damaged by the infarct at least 2, 3, 4, 5, 6, 7 days after the myocardial infarction. In one embodiment, the hydrogel is administered into the cardiac muscle damaged by the infarct within 10, 9, 8, 7, 6 or 5 days of the myocardial infarction.

In one embodiment, the hydrogel is an injectable hydrogel, and the injectable ELR hydrogel is administered into the cardiac muscle by injection.

In one embodiment, the injectable hydrogel is administered into the cardiac muscle by a method selected from epicardial injection at surgery, a minimally invasive procedure, catheter delivery percutaneously as an adjunct to intervention procedures, and as a stand-alone procedure.

In one embodiment, the hydrogel is administered (preferably injected) into a peri-infarct zone of the damaged cardiac muscle. In one embodiment, the hydrogel is administered to a plurality of sites. In one embodiment, the hydrogel is administered to a plurality of sites around the peri-infarct zone of an infarct. In one embodiment, the hydrogel is administered to a plurality of infarcts.

In one embodiment, the hydrogel is administered in an amount sufficient to modulate the response of the cardiac muscle damaged by the infarct by limiting scarring, inducing positive remodelling of the damaged muscle, inducing proliferation of small vessels, inducing a decrease in the pro-inflammatory response, and/or restoring cardiac muscle function to a clinically significant extent after infarction.

In one embodiment, the hydrogel is administered in a sufficient quantity to limit fibrosis on the cardiac muscle damaged by the infarct.

In one embodiment, the hydrogel is administered in a sufficient quantity to avoid the negative remodelling due to chronic inflammation in proximity of the cardiac muscle damaged by the infarct.

In one embodiment, the hydrogel is administered to the damaged cardiac muscle at a dosage of about 1 to about 500 µL hydrogel per Kg body weight.

In one embodiment, the hydrogel is administered to the damaged cardiac muscle at a dosage of about 10 to about 100 µL hydrogel per Kg body weight.

In one embodiment, the hydrogel is administered to the damaged cardiac muscle at a dosage of about 30 to about 100 µL hydrogel per Kg body weight.

In one embodiment, the hydrogel is administered to the damaged cardiac muscle at a dosage of about 40- to about 60 µL hydrogel per Kg body weight.

In one embodiment, the myocardial infarction is a partial (subendocardial) myocardial infarction. In one embodiment, the myocardial infarction is a full myocardial infarction (transmural).

In one embodiment, the MI is a transmural MI. In one embodiment, the MI is a non-transmural (subendocardial) MI. In one embodiment, the MI is selected from a Type I, Type 2, Type 4 or Type 5 MI.

In one embodiment, the hydrogel is chilled prior to administration, preferably about 3-5°C, and typically for a period of at least 5 minutes, for example 5-60 minutes.

In one embodiment, the hydrogel is crosslinked, preferably chemically crosslinked.

In one embodiment, the hydrogel is functionalised for chemical crosslinking with azide and/or cyclooctyne groups.

In one embodiment, the is functionalised for photo-crosslinking.

In one embodiment, the hydrogel comprises a cardioprotective agent, for example a growth factor, drug or cell. In one embodiment, the growth factor is selected from VEGF, IGF (i.e. IGF-1), eNOS, bFGF, or any subtypes thereof

In one embodiment, the hydrogel is acellular. In another embodiment, the hydrogel comprises cells. In one embodiment, the cells are stem cells. In one embodiment, the stem cells are bone marrow derived stem cells.

In one embodiment, the hydrogel is a composite hydrogel comprising an ELR and another polymer, for example a natural polymer or a synthetic polymer. Examples of natural polymers include collagen, gelatin, hyaluronic acid, fibrin, alginate, agarose, keratin, decellularized extracellular matrix (ECM). Examples of synthetic polymers include polyethylene glycol (PEG) or polyethylene oxide (PEO), or copolymers thereof, polyvinyl alcohol, and polypeptides.

In one embodiment, methods described herein further comprise adjuvant therapy, or may be combined with any form of diagnosis (e.g. history, ECG, and cardiac markers) and/or treatment (prophylactic or stabilizing treatment, such as administering oxygen, aspirin, sublingual glyceryl trinitrate, and/or pain relievers, as well as treatment with beta blockers, anticoagulation agents, ACE inhibitors, and/or antiplatelet) prior to or after administration of the hydrogel (e.g., treatment with anti-lipemic agents, anti-inflammatory agents, antithrombotic agents, fibrinolytic agents, anti-platelet agents, direct thrombin inhibitors, glycoprotein IIb/IIIa receptor inhibitors, agents that bind to cellular adhesion molecules and inhibit the ability of white blood cells to attach to such molecules (e.g. anti-cellular adhesion molecule antibodies), alpha-adrenergic blockers, beta-adrenergic blockers, cyclooxygenase-2 inhibitors, angiotensin system inhibitor, anti-arrhythmics, calcium channel blockers, diuretics, inotropic agents, vasodilators, vasopressors, thiazolidinediones, cannabinoid-1 receptor blockers and/or any combinations thereof).

In one embodiment, the method of the invention comprises administering a cardioprotective agent to the mammal, optionally as part of the hydrogel.

In another aspect, the invention provides an elastin-like recombinamer (ELR) hydrogel.

In one embodiment, the hydrogel is an injectable hydrogel.

In one embodiment, the hydrogel is crosslinked, preferably chemically crosslinked.

In one embodiment, the hydrogel is functionalised for chemical crosslinking with azide and cyclooctyne groups.

In one embodiment, the hydrogel is functionalised for photo-crosslinking.

In one embodiment, the hydrogel comprises a cardioprotective agent, for example a growth factor, drug or cell. In one embodiment, the growth factor is selected from VEGF, IGF (i.e.

IGF-1), eNOS, bFGF, or any subtypes thereof

In one embodiment, the hydrogel is acellular.

In another embodiment, the hydrogel comprises cells.

In one embodiment, the cells are stem cells.

In one embodiment, the stem cells are bone marrow derived stem cells.

In one embodiment, the hydrogel is chilled.

In one embodiment, the hydrogel is a composite hydrogel comprising an ELR and another polymer, for example a natural polymer or a synthetic polymer. Examples of natural polymers include collagen, gelatin, hyaluronic acid, fibrin, alginate, agarose, keratin, decellularized extracellular matrix (ECM). Examples of synthetic polymers include polyethylene glycol (PEG) or polyethylene oxide (PEO), or copolymers thereof, polyvinyl alcohol, and polypeptides.

Other aspects and preferred embodiments of the invention are defined and described in the other claims set out below.

### Brief Description of the Figures

**Figure 1****:** Representative pictures of the epicardial injection mode of a trial quantity of an elastin-like recombinamer hydrogel in a lamb heart collected from an abattoir. The hydrogel was stained with Alcan Blue to improve its immediate visualisation after the injection.
**Figure 2****:** Representative pictures of the physical integration of the ELR hydrogel in the tissue immediately after epicardial injection in a lamb heart collected from the abbatoir.
Tissue sections were stained with Verhoeff-van Gieson both in the area injected (right picture) with the hydrogel (right) and in the area far from the injection site as a control (left picture).
**Figure 3****:** TEM imaging of cardiomyocytes and ECM response in sheep 28 days after myocardial infarction.
   (A, B): Muscle volume fraction in the ELR hydrogel treated (a) and MI-only group (B), (C, D): Collagen volume fraction in ELR hydrogel-treated (C) and the MI-only group (D), (E, F): Masson's Trichome staining of the infarcted area in ELR hydrogel-treated (E) and control MI group (F).
   Scale bar 200 µm. n=4 t-students test *p<0.05.

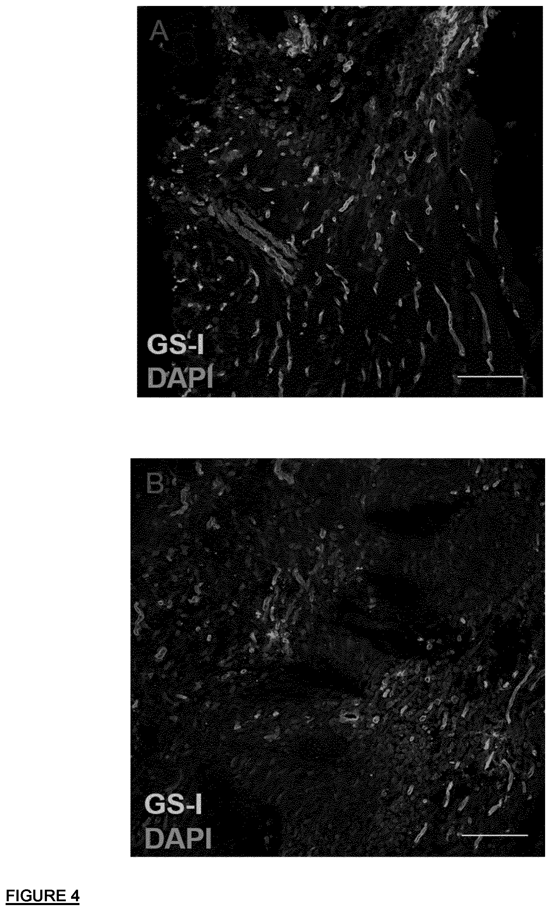

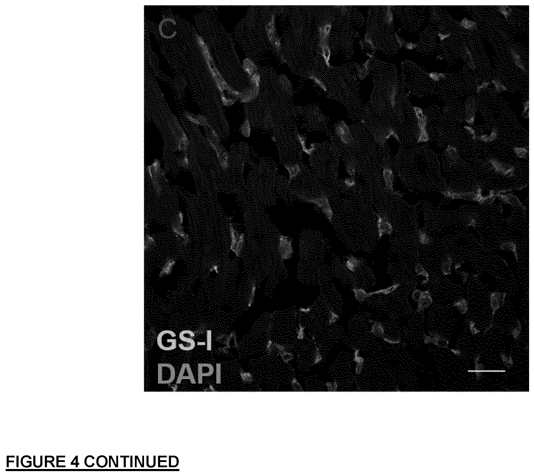

**Figure 4****:** Confocal imaging of capillaries in the ischaemic zones after ELT hydrogel injection (A) and in the MI-only control group (B) 28 days after induction of myocardial infarction. Scale bars 100 µm. Positive staining for capillary structures was validated in 5 days old neonatal Sprague-Dawley rats (C). Scale bar 20 µm.

### Detailed Description of the Invention

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "*a*" or "*an*" used in relation to an entity is to be read to refer to one or more of that entity. As such, the terms "*a*" (or "*an*"), "*one or more,*" and "*at least one*" are used interchangeably herein.

As used herein, the term "*comprise*," or variations thereof such as "*comprises*" or "*comprising*," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "*disease*" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "*treatment*" or "*treating*" refers to an intervention (e.g. the administration of an agent to a subject) which cures, ameliorates or lessens the symptoms of a disease or removes (or lessens the impact of) its cause(s) (for example, a decrease in fibrotic area, an increase muscle volume fraction in ischaemic zones, an improvement in preservation of small vessels, or a decreased pro-inflammatory response in the infarct zone). In this case, the term is used synonymously with the term "therapy".

Additionally, the terms "*treatment*" or "*treating*" refers to an intervention (e.g. the administration of an agent to a subject) which prevents or delays the onset or progression of a disease or reduces (or eradicates) its incidence within a treated population. In this case, the term treatment is used synonymously with the term "*prophylaxis*"*.*

As used herein, an "*effective amount*" or a "*therapeutically effective amount*" of an agent defines an amount that can be administered to a subject without excessive toxicity, irritation, allergic response, or other problem or complication, commensurate with a reasonable benefit/risk ratio, but one that is sufficient to provide the desired effect, e.g. a clinically significant repair of cardiac muscle evidenced by at least one or more of a clinically relevant decrease in fibrotic area, an increase muscle volume fraction in ischaemic zones, an improvement in preservation of small vessels, or a decreased pro-inflammatory response in the infarct zone, and typically within 10, 20 or 30 days of administration. The amount will vary from subject to subject, depending on the age and general condition of the individual, mode of administration and other factors. Thus, while it is not possible to specify an exact effective amount, those skilled in the art will be able to determine an appropriate "*effective*" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure. In one embodiment, the ELR hydrogel is administered to the damaged cardiac muscle at a dosage of about 1- to about 500 µL ELR hydrogel per Kg body weight. In one embodiment, the ELR hydrogel is administered to the damaged cardiac muscle at a dosage of about 10- to about 100 µL ELR hydrogel per Kg body weight. In one embodiment, the ELR hydrogel is administered to the damaged cardiac muscle at a dosage of about 30- to about 100 µL ELR hydrogel per Kg body weight. In one embodiment, the ELR hydrogel is administered to the damaged cardiac muscle at a dosage of about 40- to about 60 µL ELR hydrogel per Kg body weight.

In the context of treatment and effective amounts as defined above, the term *subject* (which is to be read to include "*individual*", "*animal*", "*patient*" or "*mammal*" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

As used herein, the term "*elastin-like recombinamer*" or "*ELR*" means a biocompatible recombinant protein-based polymer comprising a repeat sequence found in the mammalian elastic protein elastin, or a modification thereof. The most well-known members within the ELR family are based on the pentapeptide VPGVG (or its permutations), and a wide variety of polymers with the general formula (VPGXG), where X represents any natural amino acid except proline. ELR's are described in the following references:
- Rodriguez-Cabello et al (Polymer, Volume 50, Issue 22, 20 October 2009, Pages 5159-5169;
- Girotti et al (Macromolecules, 37 (9) (2004) Page 3396-3400;
- Martina et al (macromolecular Bioscience, 2 (7) (2002, Pages 319-328;
- Miao et al (Journal of Biological Chemistry, 278 (49) (2003), Pages 48553-48562;
- Zio et al (Macromolecules, 36 (5) (2003) Pages 1553-1558;
- Urry et al (Journal of Bioactive and Compatible Polymers, 6 (3) (1991) Pages 263-282;
- Mayer et al (Biomacromolecules, 3 (2) (2002) Pages 357-367;
- Spanish Patent Application No: ES2012030474; and
- European Patent Application No: 2397150.

As used herein, the term "*hydrogel*" as applied to an ELR means a three-dimensional network of ELR polymers in a water dispersion medium. In one embodiment, the ELR polymers are crosslinked (ideally chemically cross-linked) to form the three-dimensional network. Typically, the hydrogel matrix is formed of ELR homopolymer. In one embodiment, the hydrogel is injectable (i.e. has a viscosity that allows the delivery of the hydrogel in-vivo by injection). In one embodiment, the hydrogel is suitable for implantation. Methods of generating ELR hydrogels are described below and in the references above.

As used herein, the term "*cross-linked*" as applied to an ELR polymer means that the ELR polymer chains are covalently cross-linked with a crosslinking agent to form a three-dimensional network. Cross-linked ELR hydrogels are described in the literature, for example in the references above. The hydrogel of the invention may be crosslinked, crosslinkable, or not crosslinked. The hydrogel may be crosslinked prior to administration, or it may be crosslinked during or after administration. For example, the hydrogel and crosslinking agent may be administered using a syringe that keeps the two components separate until delivery where the components are mixed to allow in-situ crosslinking of the hydrogel. This may be achieved using a Duploject injection system.

As used herein, the term "*modulate the response*" as applied to cardiac muscle damaged by an infarct means one or more of a limiting scarring, inducing positive remodelling of the damaged muscle, inducing proliferation of small vessels, inducing a decrease in the pro-inflammatory response, and restoring cardiac muscle function to a clinically significant extent after infarction.

As used herein, the term "administration" in the context of a hydrogel means administration into damaged cardiac muscle, and is usually performed by means of direct injection, typically direct epicardial injection, or a catheter technique. However, other routes of administering hydrogel into the cardiac muscle may also be employed, including intravenous delivery and intraperitoneal injection. In one embodiment, the injectable hydrogel is administered into the cardiac muscle by a method selected from epicardial injection at surgery, a minimally invasive procedure, catheter delivery percutaneously (typically as an adjunct to intervention procedures), and as a stand-alone procedure.

As used herein, the term "*injectable*" as applied to an ELR hydrogel means that the ELR hydrogel can be epicardially injected into heart muscle of a mammal. The term "epicardial injection" means injection into the cardiac muscle through the epicardium, the envelope of tissue that surrounds the heart of mammals. In one embodiment, the injection is carried out using a 25G syringe. Various systems and methods are available for direct injection into the heart, including the EpiAccess System (EpiEP, New Haven, CT, USA).

As used herein, the term "*peri-infarct zone*" means the periphery of an area of infarct that separates the ischaemic cardiac muscle and surrounding healthy non-ischaemic cardiac muscle. In one embodiment, the treatment comprises administering ELR hydrogel at a plurality of locations around the peri-infarct zone, for example 2-10 injections, and preferably at least 3, 4 or 5.

As used herein, the term "*anti-fibrosis effects*" means a reduction in the area of fibrosis is the treated cardiac muscle.

As used herein, the term "*anti-inflammatory effects*" means a reduction in the level of pro-inflammatory mediators (e.g. proinflammatory cytokines) present in the treated cardiac muscle.

As used herein, the term "*partial myocardial infarction*" means a myocardial infarction characterised by a partial blockage of a coronary artery, otherwise referred to as subendocardial or non-ST-elevation MI (NSTEMI). As used herein, the term "*full myocardial infarction*" means a myocardial infarction characterised by a full blockage of a coronary artery otherwise referred to as ST-elevation MI (STEMI) or transmural infarction. Determining whether a myocardial infarction is partial or full can be determined by suitable imaging, for example a cardiac MRI scan, echocardiogram or interpretation of an ECG.

As used herein, the term "*Type 1*" myocardial infarction" means a spontaneous MI related to ischemia from a primary coronary event (e.g., plaque rupture, thrombotic occlusion). "*Type 2*" myocardial infarction is secondary to ischemia from a supply-and-demand mismatch. "*Type 3*" myocardial infarction is an MI resulting in sudden cardiac death. "*Type 4a*" myocardial infarction is an MI associated with percutaneous coronary intervention, and *"Type 4b"* is associated with in-stent thrombosis. "*Type 5*" is an MI associated with coronary artery bypass surgery.

As used herein, the term "*transmural myocardial infarction*" means a myocardial infarction characterised by ischaemic necrosis of the full thickness of the affected muscle segments, extending from the endocardium through the myocardium to the epicardium. A "*non-transmural myocardial infarction*" is defined as an area of ischemic necrosis that does not extend through the full thickness of myocardial wall segment(s). In a non-transmural MI (subendocardial or NSTEMI), the area of ischemic necrosis is limited to the endocardium or to the endocardium and myocardium. It is the endocardial and subendocardial zones of the myocardial wall segment that are the least perfused regions of the heart and the most vulnerable to conditions of ischemia.

As used herein, the term "*cardioprotective agents*" refers to agents that exert certain effects, such as anti-apoptotic, anti-necrotic/cell death, anti-inflammatory, anti-fibrotic, and/or regenerative effects, that are beneficial to the patient or subject suffering from an acute myocardial infarction. Cardioprotective agents of the invention may exert their beneficial effects on cells such as for example myocytes in tissues such as heart tissues, but may also exert their effects on cells such as epithelial or endothelial cells present for example in arteries supplying the heart. When supplied sufficiently early after the occurrence of myocardial infarction the beneficial effects may include a reduction or prevention of cell death, such as through necrosis or apoptosis, of myocytes at the periphery of developing myocardial infarcts and/or an induction of growth of surviving myocytes. The cardioprotective agents may also stimulate growth or prevent cell death of epithelial or endothelial cells present in arteries supplying the heart. These effects may lead to a reduction in size and/or partial or complete restoration of the moderately injured and/or necrotic tissue of the infarcted area. Additional beneficial effects may include dissolving blood clots or other organic material leading to arterial constrictions or occlusions, such as vulnerable atherosclerotic plaques. Cardioprotective agents disclosed herein that exert anti-apoptotic effects include TGFβ1, IGF1, eNOS and bFGF and its subtypes. Other cellular factors that are involved in tissue repair or maintenance, stem cell factors, anti-apoptotic factors and/or growth factors may also be useful as cardioprotective agents. Such agents include granulocyte colony stimulating factor (GCSF), platelet-derived growth factor (PDGF), vascular endothelial growth factor (VEGF), fibroblast growth factors (FGFs), Angiotensin II, and stromal cell-derived factor 1 (SDF-1). TGF-β is a secreted protein that exists in three isoforms TGF-β1, TGF-β2 and TGF-β3. The TGF-β family is part of a superfamily of proteins known as the transforming growth factor beta superfamily, which includes inhibins, activin, anti-mullerian hormone, bone morphogenetic protein, decapentaplegic and Vg-1. The insulin-like growth factors (IGFs) are polypeptides with high sequence similarity to insulin and comprise of cell-surface receptors (e.g. IGF1R and IGF2R), ligands (e.g. IGF-1 and IGF-2), IGF binding proteins (e.g. IGFBP 1-6), as well as associated IGFBP proteases. Insulin-like growth factor 1 (IGF-1) is mainly secreted by the liver as a result of stimulation by growth hormone (GH) and plays a role in the promotion of cell proliferation and the inhibition of cell death (apoptosis). Insulin-like growth factor 2 (IGF-2) is thought to be a primary growth factor required for early development while IGF-1 expression is required for achieving maximal growth. Many cardioprotective agents disclosed herein, such as TGFβ1 and IGF1, are commercially available in purified or recombinant form and have been suggested for many therapeutic uses, such as for example wound healing (U.S. Patent Nos. 4,861,757; 4,983,581 and 5,256,644), or induction of bone growth (U.S. Patent No. 5,409,896 and 5,604,204).

### Exemplification

The invention will now be described with reference to specific Examples. These are merely exemplary and for illustrative purposes only: they are not intended to be limiting in any way to the scope of the monopoly claimed. These examples constitute the best mode currently contemplated for practicing the invention.

### Hydrogel preparation and injection conditions

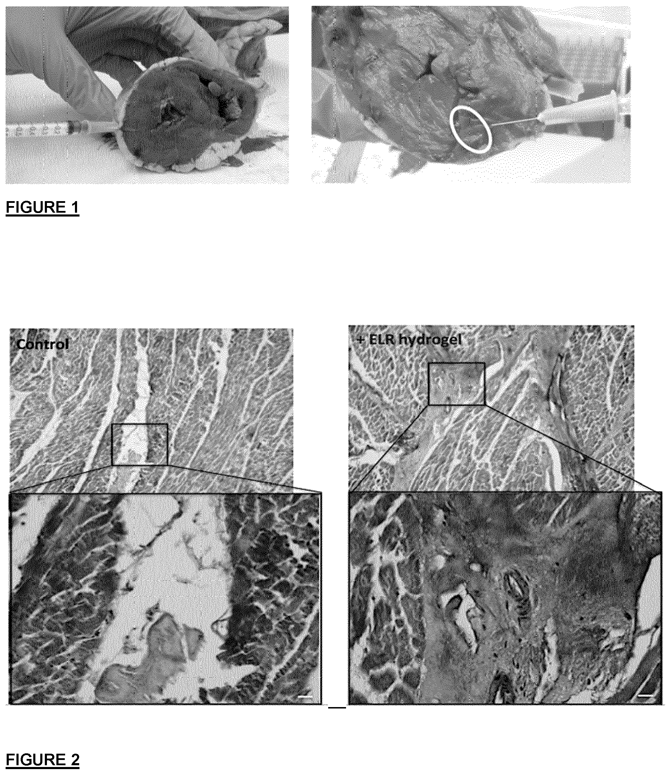

Elastin-like recombinamers (ELRs) functionalised with azide (HRGD6) and cyclooctyne (HE5) groups for crosslinking were weighed under sterile conditions. ELRs were resuspended to maintain the HE5-cycloctyne to HRGD6-azide ratio at 1.79:1. ELRs were stored at 4°C until the day before they were required for injection. Therefore, each ELR was dissolved in 750 µl of sterile molecular grade water overnight at 4°C. Once the solutions were totally clear, 250 µl of HRGD6-azide was placed in a sterile eppendorf tube, where 250 µl of HE5-cycloctyne was added to initiate crosslinking of the recombinamers and hydrogel formation. Cold sterile tips were used to carry out this procedure and the eppendorf tube containing the mixed ELRs was kept on ice for exactly 10 minutes before injection. The feasibility of the ELRs hydrogel epicardial injection and its physical integration in the myocardial tissue was tested ex vivo in a lamb heart collected from the abattoir (Figure 1 and Figure 2).

### Large Animal (Sheep) In Vivo Study

Myocardial infarction was induced in five months old male sheep (weight 30 kg) by transmural suture ligation at intervals parallel to and along the left anterior descending artery. This novel concept was developed to mimic as closely as possible, the real-life type of myocardial infarction seen in clinical practice, unlike previous models of simple LAD ligation. Cardiothoracic surgeon (Mark Da Costa) and the veterinary team ensured that the surgical procedure was performed according to current European animal laboratory guidelines and practice. One week after MI induction the animal was re-operated and the hydrogel solution was injected into the peri-infarct zone by the repeated administration of 500 µl of ELR solution by epicardial injection. A sterile syringe having a maximum capacity of 1 ml and a 25G needle was used to perform each epicardial injection through the wall of the heart. Three hydrogels of 500 µl volume each were injected per sheep through the peri-infarct zone, which is the edge of the region between the site where ischemia was induced by coronary ligation and the healthy zone of the myocardial walls. The needle was carefully retracted after each injection to avoid backflow of the hydrogel solution. Echocardiography measurements were performed before surgery and at day 28 days post MI induction and 21 days post injection of the hydrogel.

### Histological analysis

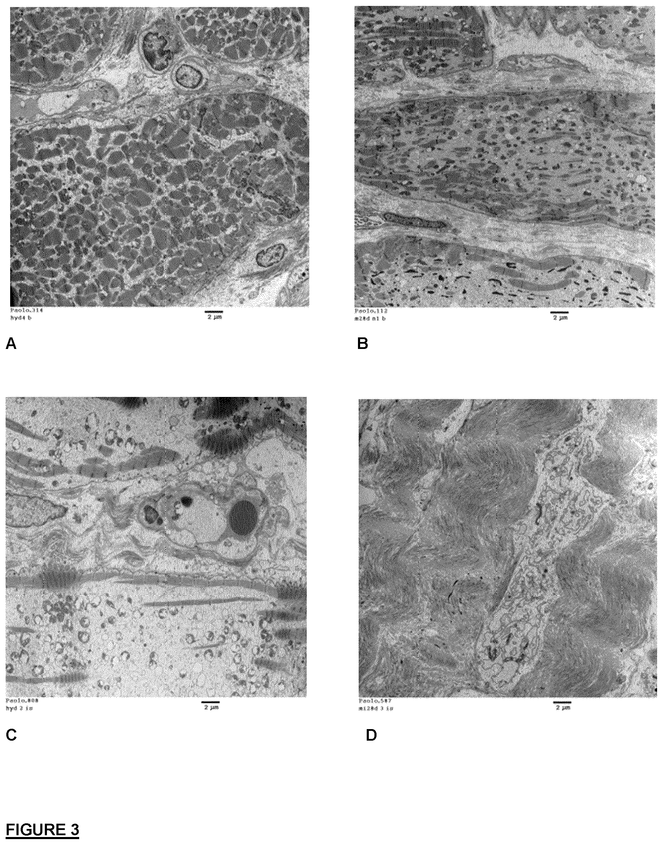

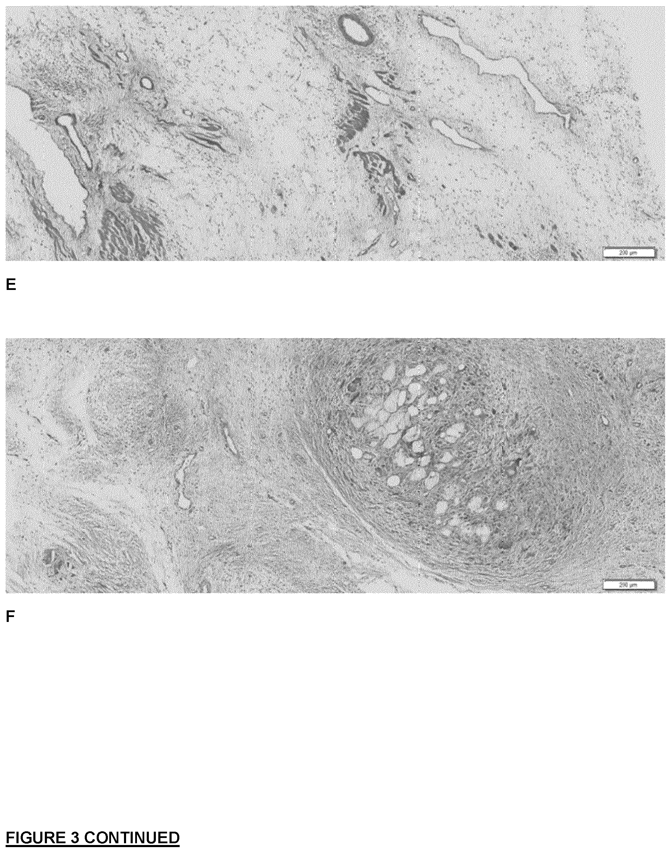

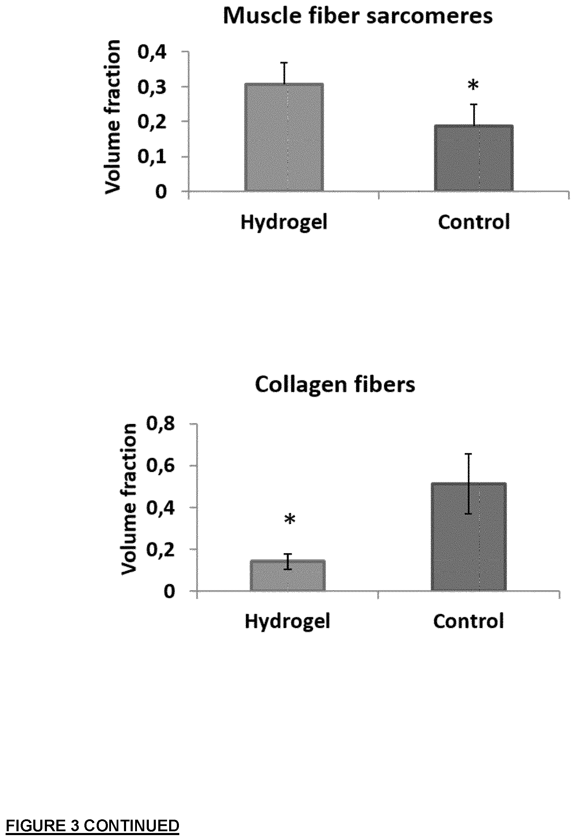

According to our first preliminary analysis on a first batch of animals, the hydrogel-treated group showed a decreased fibrotic area and an increased muscle volume fraction in the ischemic zones by TEM imaging (Figure 3 (c) Figure 3 (d)). Masson's Trichrome staining confirmed the deposition of collagen in the fibrotic areas which was quantified to validate the first set of analysis once the harvesting of all the tissue was completed (Figure 3(e); Figure 3(f)). The preservation of small vessels was observed in both the elastin treated group and the control group by immunofluorescence experiments for CD31 and GS-I lectin markers. Specifically GS-I staining for capillaries and endothelial structures - which had been validated as a control in neonatal rat myocardial tissue - showed a higher expression in the ELR hydrogel treated animals (Figure 4).

### Equivalents

The foregoing description details presently preferred embodiments of the present invention. Numerous modifications and variations in practice thereof are expected to occur to those skilled in the art upon consideration of these descriptions. Those modifications and variations are encompassed within the claims appended hereto.

## Claims

1. An injectable elastin-like recombinamer (ELR) hydrogel, for use in a method of treating a mammal that has suffered a myocardial infarction to modulate the response of cardiac muscle damaged by the infarct, in which the hydrogel is injected into the cardiac muscle damaged by the infarct at least two days after the myocardial infarction, and in an amount sufficient to modulate the response of cardiac muscle damaged by the infarct.

2. An ELR hydrogel according to Claim 1, for use of Claim 1, in which the hydrogel is injected into the cardiac muscle damaged by the infarct at least three days after the myocardial infarction,

3. An ELR hydrogel according to Claim 1, for use of Claim 1, in which the hydrogel is injected into the cardiac muscle damaged by the infarct about 2 to 7 days after the myocardial infarction,

4. An ELR hydrogel according to Claim 1, for use of Claim 1, 2 or 3, in which the myocardial infarction is a partial myocardial infarction.

5. An ELR hydrogel according to Claim 1, for use of any of Claims 1 to 4, for limiting scarring, inducing positive remodelling of the damaged muscle, and/or restoring cardiac muscle function to a clinically significant extent after infarction.

6. An ELR hydrogel according to Claim 1, for use of any of Claims 1 to 5, in which the ELR hydrogel is injected into a peri-infarct zone of the damaged cardiac muscle.

7. An ELR hydrogel according to Claim 1, for use of any of Claims 1 to 6, in which the ELR hydrogel is injected into a plurality of spaced-apart sites of the damaged cardiac muscle.

8. An ELR hydrogel according to Claim 1, for use of any of Claims 1 to 7, in which the ELR hydrogel is administered to the damaged cardiac muscle at a dosage of 1-500 µL ELR hydrogel per Kg body weight.

9. An ELR hydrogel according to Claim 1, for use of any of Claims 1 to 8, in which the ELR hydrogel is administered to the damaged cardiac muscle at a dosage of 10-100 µL ELR hydrogel per Kg body weight.

10. An ELR hydrogel according to Claim 1, for use of any of Claims 1 to 9, in which the ELR hydrogel is chilled prior to administration.

11. An ELR hydrogel according to Claim 1, for use of any of Claims 1 to 10, in which the ELR hydrogel is chemically crosslinked.

12. An ELR hydrogel according to Claim 1, for use of any of Claims 1 to 11, in which the ELR is functionalised with azide and cyclooctyne groups.

13. An ELR hydrogel according to Claim 1, for use of any of Claims 1 to 11, in which the ELR is functionalised with glycan moieties.

## Patentansprüche

1. Injizierbares Hydrogel von Elastin-ähnlichem Rekombinamer (ELR) für die Verwendung in einem Verfahren zur Behandlung eines Säugers, der einen Myokardinfarkt erlitten hat, um die Reaktion von Herzmuskel, der durch den Infarkt geschädigt ist, zu modulieren, in dem das Hydrogel in den Herzmuskel, der durch den Infarkt geschädigt ist, mindestens zwei Tage nach dem Myokardinfarkt und in einer Menge injiziert wird, die ausreicht, um die Reaktion von Herzmuskel, der durch den Infarkt geschädigt ist, zu modulieren.

2. ELR-Hydrogel nach Anspruch 1 für die Verwendung nach Anspruch 1, in dem das Hydrogel in den Herzmuskel, der durch den Infarkt geschädigt ist, mindestens drei Tage nach dem Myokardinfarkt injiziert wird.

3. ELR-Hydrogel nach Anspruch 1 für die Verwendung nach Anspruch 1, in dem das Hydrogel in den Herzmuskel, der durch den Infarkt geschädigt ist, etwa 2 bis 7 Tage nach dem Myokardinfarkt injiziert wird.

4. ELR-Hydrogel nach Anspruch 1 für die Verwendung nach Anspruch 1, 2 oder 3, in dem der Myokardinfarkt ein partieller Myokardinfarkt ist.

5. ELR-Hydrogel nach Anspruch 1 für die Verwendung nach einem der Ansprüche 1 bis 4 für die Begrenzung von Narbenbildung, Auslösung von positiver Umbildung des geschädigten Muskels und/oder Wiederherstellung von Herzmuskelfunktion in einem klinisch signifikanten Maße nach dem Infarkt.

6. ELR-Hydrogel nach Anspruch 1 für die Verwendung nach einem der Ansprüche 1 bis 5, in dem das ELR-Hydrogel in eine Periinfarktzone des geschädigten Herzmuskels injiziert wird.

7. ELR-Hydrogel nach Anspruch 1 für die Verwendung nach einem der Ansprüche 1 bis 6, in dem das ELR-Hydrogel in eine Vielzahl von beabstandeten Stellen des geschädigten Herzmuskels injiziert wird.

8. ELR-Hydrogel nach Anspruch 1 für die Verwendung nach einem der Ansprüche 1 bis 7, in dem das ELR-Hydrogel an den geschädigten Herzmuskel in einer Dosierung von 1-500 µl ELR-Hydrogel pro kg Körpergewicht verabreicht wird.

9. ELR-Hydrogel nach Anspruch 1 für die Verwendung nach einem der Ansprüche 1 bis 8, in dem das ELR-Hydrogel an den geschädigten Herzmuskel in einer Dosierung von 10-100 µl ELR-Hydrogel pro kg Körpergewicht verabreicht wird.

10. ELR-Hydrogel nach Anspruch 1 für die Verwendung nach einem der Ansprüche 1 bis 9, in dem das ELR-Hydrogel vor der Verabreichung gekühlt ist.

11. ELR-Hydrogel nach Anspruch 1 für die Verwendung nach einem der Ansprüche 1 bis 10, in dem das ELR-Hydrogel chemisch vernetzt ist.

12. ELR-Hydrogel nach Anspruch 1 für die Verwendung nach einem der Ansprüche 1 bis 11, in dem das ELR mit Azid- und Cyclooctingruppen funktionalisiert ist.

13. ELR-Hydrogel nach Anspruch 1 für die Verwendung nach einem der Ansprüche 1 bis 11, in dem das ELR mit Glycaneinheiten funktionalisiert ist.

## Revendications

1. Hydrogel de recombinamère de type élastine (ELR) injectable, pour une utilisation dans une méthode destinée au traitement d'un mammifère ayant souffert d'un infarctus du myocarde afin de moduler la réponse du muscle cardiaque endommagé par l'infarctus, l'hydrogel étant injecté dans le muscle cardiaque endommagé par l'infarctus au moins deux jours après l'infarctus du myocarde, et selon une quantité suffisante pour moduler la réponse du muscle cardiaque endommagé par l'infarctus.

2. Hydrogel d'ELR selon la revendication 1, pour une utilisation selon la revendication 1, l'hydrogel étant injecté dans le muscle cardiaque endommagé par l'infarctus au moins trois jours après l'infarctus du myocarde.

3. Hydrogel d'ELR selon la revendication 1, pour une utilisation selon la revendication 1, l'hydrogel étant injecté dans le muscle cardiaque endommagé par l'infarctus environ 2 à 7 jours après l'infarctus du myocarde.

4. Hydrogel d'ELR selon la revendication 1, pour une utilisation selon la revendication 1, 2 ou 3, l'infarctus du myocarde étant un infarctus du myocarde partiel.

5. Hydrogel d'ELR selon la revendication 1, pour une utilisation selon l'une quelconque des revendications 1 à 4, pour limiter la cicatrisation, induire un remodelage positif du muscle endommagé et/ou restaurer la fonction du muscle cardiaque dans une mesure cliniquement significative après un infarctus.

6. Hydrogel d'ELR selon la revendication 1, pour une utilisation selon l'une quelconque des revendications 1 à 5, l'hydrogel d'ELR étant injecté dans une zone péri-infarctus du muscle cardiaque endommagé.

7. Hydrogel d'ELR selon la revendication 1, pour une utilisation selon l'une quelconque des revendications 1 à 6, l'hydrogel d'ELR étant injecté dans une pluralité de sites espacés les uns des autres du muscle cardiaque endommagé.

8. Hydrogel d'ELR selon la revendication 1, pour une utilisation selon l'une quelconque des revendications 1 à 7, l'hydrogel d'ELR étant administré au muscle cardiaque endommagé selon un dosage de 1-500 µl d'hydrogel d'ELR par kg de poids corporel.

9. Hydrogel d'ELR selon la revendication 1, pour une utilisation selon l'une quelconque des revendications 1 à 8, l'hydrogel d'ELR étant administré au muscle cardiaque endommagé selon un dosage de 10-100 µl d'hydrogel d'ELR par kg de poids corporel.

10. Hydrogel d'ELR selon la revendication 1, pour une utilisation selon l'une quelconque des revendications 1 à 9, l'hydrogel d'ELR étant refroidi préalablement à l'administration.

11. Hydrogel d'ELR selon la revendication 1, pour une utilisation selon l'une quelconque des revendications 1 à 10, l'hydrogel d'ELR étant réticulé par voie chimique.

12. Hydrogel d'ELR selon la revendication 1, pour une utilisation selon l'une quelconque des revendications 1 à 11, l'ELR étant fonctionnalisé par des groupements azoture et cyclooctyne.

13. Hydrogel d'ELR selon la revendication 1, pour une utilisation selon l'une quelconque des revendications 1 à 11, l'ELR étant fonctionnalisé par des motifs glycane.
